# EUROPEAN PATENT APPLICATION

(11) **EP 4 643 932 A1**
(43) Date of publication of application: **05.11.2025**
(21) Application number: 23912790.5
(22) Date of filing: 21.12.2023
(51) Int. Cl.: A61N 1/36, A61N 1/40, A61N 1/32, A61N 1/06, A61N 5/06, A61N 1/08

(54) **COSMETIC PROCEDURE DEVICE HAVING CONTACT DETECTION SENSOR, AND CONTROL METHOD THEREFOR**

(30) Priority: 28.12.2022 KR 20220186645
(71) Applicant: APR CO., LTD., Seoul 05551 (KR)
(72) Inventor: JI, Jong Chul, Seoul 08592 (KR); LEE, Gyoun Jung, Seoul 08592 (KR); PARK, Seung Woo, Seoul 08592 (KR)
(74) Representative: Locas, Davide
(86) International application number: PCT/KR2023/021316
(87) International publication number: WO 2024/144115

(57) **Abstract**

A beauty treatment device is disclosed. A beauty treatment device according to one aspect of the present disclosure is a beauty treatment device for performing a beauty treatment on a skin of a user, the beauty treatment device including: a main body having a contact surface that comes into contact with the skin of the user; a stimulation application unit provided inside the main body to apply stimulation to the skin of the user in a state where the contact surface is in contact with the skin of the user; a contact detection unit including a contact detection sensor provided on the contact surface; and a controller for determining whether the contact detection sensor is in contact with the skin of the user and controlling the stimulation application unit so that the stimulation application unit operates only when the contact detection sensor is in contact with the skin of the user.

## Description

### TECHNICAL FIELD

The present disclosure relates to a beauty treatment device and a control method therefor, and more specifically, to a beauty treatment device having a contact detection sensor capable of detecting whether a main body of the beauty treatment device has come into contact with a skin of a user, and a control method therefor.

### BACKGROUND ART

Recently, the distribution of home beauty treatment devices that allow users to perform beauty treatments such as massage and hair removal at home has been increasing. Home beauty treatment devices allow users to perform beauty treatments easily at their own home at their own desired time, thereby lowering the barrier to beauty treatment and increasing accessibility.

As the spread of home beauty treatment devices increases, the performance of home beauty treatment devices is also improving dramatically compared to the past, and the effectiveness of the treatment is also increasing. This means that when the user malfunctions the device or does not use the device safely and appropriately, the possibility of side effects occurring and the degree of side effects may also increase.

Specifically, home beauty treatment devices perform hair removal, massage, or the like using lasers, radiofrequency, or the like. When applying radiofrequency to the skin for beauty and treatment, the high-frequency electrode and the skin should be in perfect contact to generate deep heat along with the appropriate treatment effect.

However, when radiofrequency is applied in a state where a contact area with the skin is small due to a contact angle between the main body of the beauty treatment device and the skin, pressure fluctuations, or skin curvature, a current density increases, which poses a risk of burns.

Accordingly, as the performance and effectiveness of home beauty treatment devices have improved, there is a need to enable skin stimulation only when the contact surface of the main body of the beauty treatment device touches the skin to prevent risks due to incorrect or unintended use of the product.

### DISCLOSURE

### TECHNICAL PROBLEM

The present disclosure is to solve the above problems, and an object of the present disclosure is to provide a beauty treatment device and a control method therefor that enable a stimulation application unit to operate only when a main body of the beauty treatment device is in contact with skin.

Another object of the present disclosure is to provide a beauty treatment device and a control method therefor that may accurately determine whether the main body of the beauty treatment device is in close contact with the skin.

Another object of the present disclosure is to provide a beauty treatment device and a control method therefor that may be safely used by a user by allowing the stimulation application unit to apply stimulation to the skin only when the main body of the beauty treatment device is in contact with the skin.

Another object of the present disclosure is to provide a beauty treatment device and a control method therefor that may reduce possibility of a burn due to the contact surface between the skin and the beauty treatment device not being in close contact with each other when the beauty treatment device generates radiofrequency to apply stimulation to the skin.

Objects of the present disclosure are not limited to the objects mentioned above, and other objects not mentioned can be clearly understood by those skilled in the art to which the present disclosure belongs from the description below.

### TECHNICAL SOLUTION

According to one aspect of the present disclosure, there is provided a beauty treatment device having a contact detection sensor to perform a beauty treatment on skin of a user, the beauty treatment device including: a main body having a contact surface that comes into contact with the skin of the user; a stimulation application unit provided inside the main body to apply stimulation to the skin of the user in a state where the contact surface is in contact with the skin of the user; a contact detection unit including a contact detection sensor provided on the contact surface; and a controller for determining whether the contact detection sensor is in contact with the skin of the user and controlling the stimulation application unit so that the stimulation application unit operates only when the contact detection sensor is in contact with the skin of the user.

In this case, the stimulation applied to the skin of the user by the stimulation application unit may be provided by a high-frequency electrode or light emitted to the skin.

In this case, the contact surface may be formed as one of a circle, an ellipse, and a polygon, the stimulus applied from the stimulation application unit may be formed to be applied toward the skin of the user at inside the contact surface or at a location on a periphery of the contact surface, and the contact detection sensor may be positioned at a different location on the periphery of the contact surface.

In this case, the contact detection sensor may include at least one pair of contact detection members disposed on the periphery of the contact surface.

In this case, the contact detection member may be disposed to be spaced apart at equal intervals along the periphery of the contact surface.

In this case, the contact detection unit may include: a capacitor electrically connected to the contact detection member and having a capacitance that varies depending on whether or not there is contact with the skin; a voltage supply module that supplies a predetermined square wave voltage to the capacitor; and an AD converter for measuring the capacitance of the capacitor.

In this case, the contact detection member may include first and second contact detection members that are spaced apart from each other, the capacitor may be electrically connected to the voltage supply module and connected to the first contact detection member, and a ground may be connected in parallel to the capacitor when both the first contact detection member and the second contact detection member come into contact with the skin of the user.

In this case, the beauty treatment device may further include a switching element for turning on and off the square wave voltage supplied from the voltage supply module.

In this case, wherein the controller may determine that the skin is not in contact when it is determined that the capacitance according to the predetermined square wave voltage supplied from the voltage supply module is a specific value, and may determine that the skin is in contact when the capacitance is not the specific value.

In this case, the controller may provide a first time interval signal for applying the stimulus from the stimulation application unit to the stimulation application unit and provide a second time interval signal for supplying the square wave voltage from the voltage supply module to the voltage supply module, and the first time interval signal and the second time interval signal may be alternately and periodically provided.

According to another aspect of the present disclosure, there is provided a control method for a beauty treatment device having a contact detection sensor including a stimulation application unit that applies stimulation to skin of a user by coming into contact with the skin of the user and a capacitive sensor for detecting whether the device has come into contact with the skin of the user, the control method including: first determining whether a main body of the beauty treatment device has come into contact with the skin; applying stimulation to the skin for a first time interval from the stimulation application unit when the main body has come into contact with the skin; providing a square wave voltage to measure a capacitance of the capacitive sensor of a contact detection unit for a second time interval after the first time interval has elapsed; and second determining whether the main body has come into contact with the skin.

In this case, in the determining of whether the main body of the beauty treatment device is in contact with the skin, when the capacitance of the capacitive sensor corresponds to a specific value corresponding to an output value of the square wave voltage, it may be determined that the main body is not in contact with the skin, and when the capacitance does not correspond to the specific value, it may be determined that the main body is in contact with the skin.

In this case, after the applying of the stimulus to the skin during the first time interval and the providing of the square wave voltage to measure the capacitance of the capacitive sensor of the contact detection unit during the second time interval after the first time interval has elapsed are repeated multiple times, the second determining of whether the main body has come into contact with the skin may be performed.

### ADVANTAGEOUS EFFECTS

According to the above-described configuration, the beauty treatment device according to one embodiment of the present disclosure has the contact detection member of the contact detection unit on the skin contact surface of the main body of the skin beauty device so as to confirm whether the contact surface of the skin beauty device is in close contact with the skin.

The beauty treatment device according to one embodiment of the present disclosure is configured to apply stimulation to the skin only when the contact surface of the beauty treatment device is in close contact with the skin, thereby preventing in advance any burns or unpredictable accidents that may occur due to the beauty treatment device not being in close contact with the skin, thereby allowing the user to use the skin beauty device safely.

In addition, in the beauty treatment device, according to one embodiment of the present disclosure, when the skin beauty device is used for the first time, the contact detection unit first determines whether or not it is in contact with the skin, and when the stimulation application unit is operated, the stimulation application unit and the contact detection unit are operated alternately, so that even when the skin beauty device is separated from the skin while the stimulation application unit is in operation, the contact detection unit can secondarily confirm a non-contact state. Therefore, the stimulation application unit can stop operating in a state where the skin beauty device is separated from the skin, and thus, the user can safely use the skin beauty device.

Effects of the present disclosure are not limited to the effects described above, and should be understood to include all effects that can be inferred from the composition of the disclosure described in the detailed description or claims of the present disclosure.

### DESCRIPTION OF DRAWINGS

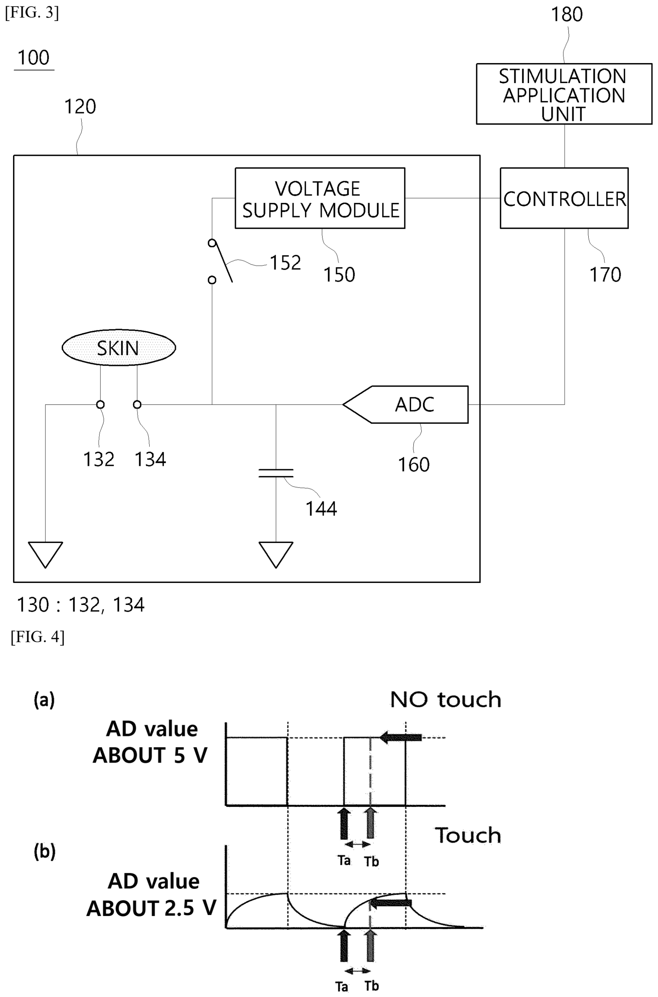

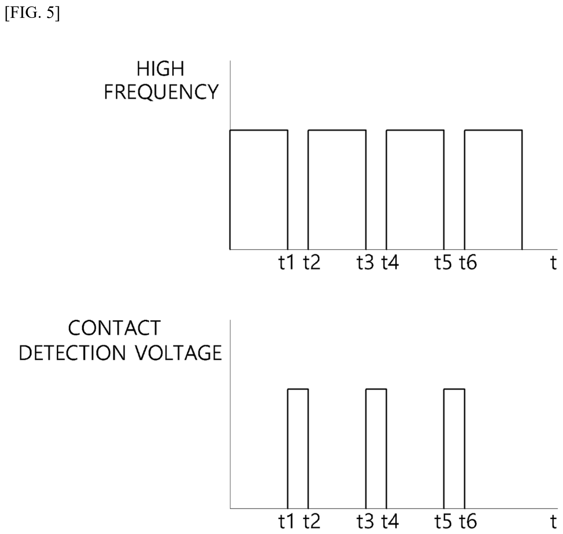

FIG. 1 is a perspective view of a skin beauty device according to one embodiment of the present disclosure viewed from above.
FIG. 2 is a perspective view of the skin beauty device according to one embodiment of the present disclosure viewed from below.
FIG. 3 is a schematic configuration diagram of the skin beauty device according to one embodiment of the present disclosure.
(a) of FIG. 4 is a graph illustrating a case where it is determined that a capacitance according to voltage supplied from a voltage supply module of the skin beauty device according to one embodiment of the present disclosure corresponds to a specific value and thus no contact has been made with the skin, and (b) of FIG. 4 is a graph illustrating a case where it is determined that the capacitance according to the voltage supplied from the voltage supply module of the skin beauty device according to one embodiment of the present disclosure does not correspond to the specific value and thus contact has been made with the skin.
(a) of FIG. 5 is a graph illustrating a time interval during which a stimulation application unit applies a stimulation in the skin beauty device according to one embodiment of the present disclosure, and (b) of FIG. 5 is a graph illustrating a time interval during which the voltage supply module supplies a contact detection voltage.

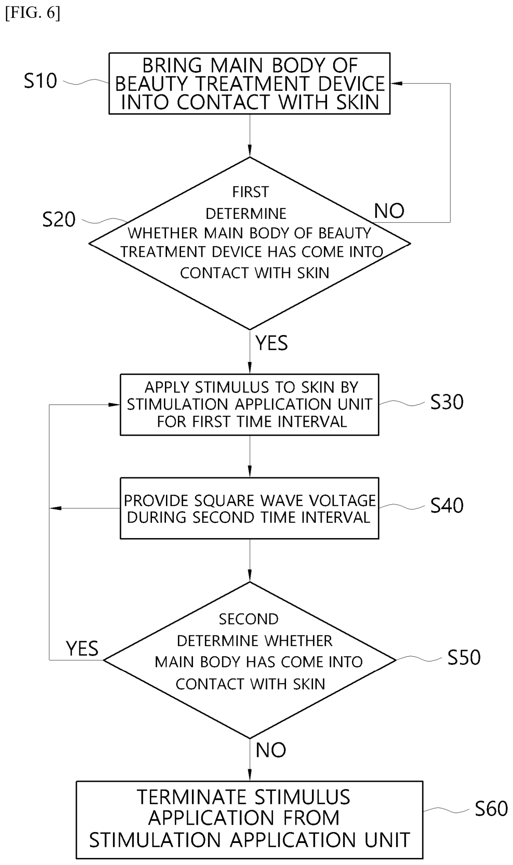

FIG. 6 is a flowchart illustrating a method for controlling a skin beauty device according to one embodiment of the present disclosure.

100: Beauty treatment device
110: Main body
120: Contact detection unit
132: First contact detection member
134: Second contact detection member
144: Capacitor
150: Voltage supply module
160: AD converter
170: Controller
180: Stimulation application unit

### BEST MODE

Hereinafter, with reference to the attached drawings, embodiments of the present disclosure will be described in detail so that those with ordinary skill in the art can easily implement the present disclosure. The present disclosure may be implemented in various different forms and is not limited to the embodiments described herein. In order to clearly describe the present disclosure, parts that are not related to the description are omitted in the drawings, and the same reference numerals are assigned to the same or similar components throughout the specification.

The words and terms used in the present specification and claims should not be construed as limited to their usual or dictionary meanings, but should be interpreted as meanings and concepts consistent with the technical idea of the present disclosure according to the principles by which the inventor can define terms and concepts in order to best explain his or her disclosure.

Therefore, the embodiments described in the present specification and the configurations illustrated in the drawings correspond to a preferred embodiment of the present disclosure and do not represent all of the technical ideas of the present disclosure, so the corresponding configuration may have various equivalents and modified examples that can replace it at the time of filing of the present disclosure.

In the present specification, the terms "include" or "have" are intended to describe the presence of a feature, number, step, operation, component, part or combination therefor described in the specification, but should be understood as not excluding in advance the possibility of the presence or addition of one or more other features, numbers, steps, operations, components, parts or combinations therefor.

When a component is said to be "in front", "behind", "above" or "below" another component, unless there are special circumstances, it includes not only being disposed "in front", "behind", "above" or "below" the other component in direct contact with it, but also if there is another component in between. Furthermore, when a component is said to be "connected" to another component, unless there are special circumstances, it includes not only being directly connected to one another, but also being indirectly connected to one another.

Hereinafter, a beauty treatment device having a contact detection sensor according to one embodiment of the present disclosure will be described with reference to the drawings.

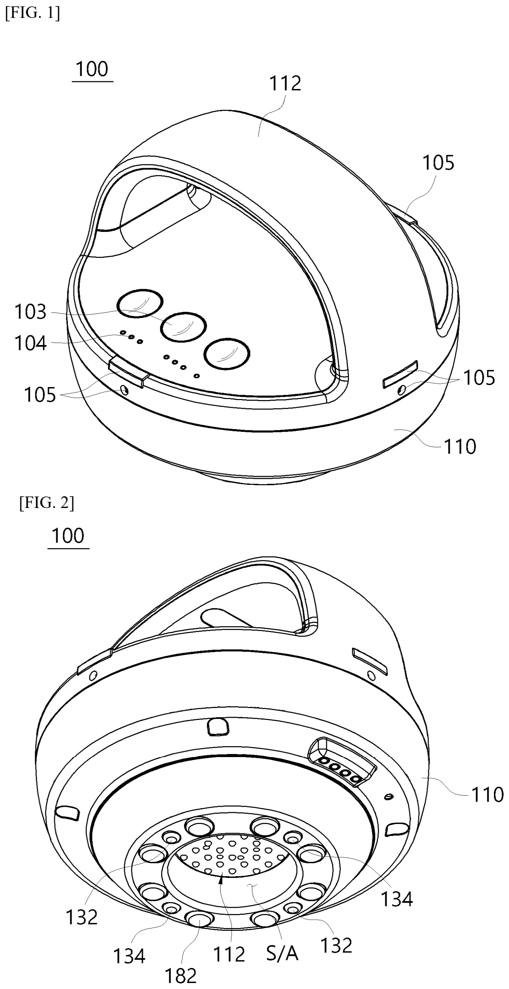

FIG. 1 is a perspective view of a skin beauty device according to one embodiment of the present disclosure as viewed from above. FIG. 2 is a perspective view of the skin beauty device according to one embodiment of the present disclosure as viewed from below.

Referring to FIGS. 1 and 2, a beauty treatment device 100 having a contact detection sensor according to one embodiment of the present disclosure is a beauty treatment device that allows a user to perform a beauty treatment on his or her own skin. Meanwhile, a user may also perform a beauty treatment on a recipient using the beauty treatment device.

For example, the beauty treatment device 100 according to one embodiment of the present disclosure may provide a suction function that stimulates the skin of a user or a recipient by sucking the skin toward the beauty treatment device using a stimulation application unit installed in the beauty treatment device, provide a massage to the skin using a high-frequency electrode, or apply stimulation such as laser hair removal.

Components for the beauty treatment may be disposed in a main body 110. For example, electrodes for high-frequency stimulation or a light source (not illustrated) for laser or light irradiation may be installed in the main body 110. In addition, components (not illustrated) for providing the suction function that sucks in and stimulates the skin of the user may also be disposed in the main body 110.

Referring to FIG. 1, a handle portion 112 may be provided on the upper side of the main body 110 so that the user can hold and move the beauty treatment device 100.

Moreover, referring to FIG. 1, the beauty treatment device 100 according to one embodiment of the present disclosure may be equipped with an operation button 103 for receiving operation information for turning the beauty treatment device 100 on/off, selecting various modes, or the like. This is exemplary, and instead of the operation button 103, a touch screen or the like may be provided on the surface of the main body.

Meanwhile, the beauty treatment device 100 according to one embodiment of the present disclosure may be equipped with an output unit 104 for outputting a signal, message, or the like. In this case, the output unit 104 may be a speaker for outputting sound or voice. In addition, the output unit 104 may be a display for displaying visual information.

In addition, a display unit 105 for indicating the direction of movement of the skin beauty device may be provided around the main body. The display unit 105 may be provided as a blinking optical element for indicating the direction of movement of the skin beauty device, and the user may operate the skin beauty device according to the display of such an optical element.

Meanwhile, according to one embodiment of the present disclosure, the main body 110 of the beauty treatment device has a contact surface 112 at the lower portion that can come into contact with a predetermined area of the skin of the user.

In the present embodiment, the contact surface 112 of the lower portion of the main body of the beauty treatment device is formed in a circular shape. In this case, the contact surface 112 is an area that comes into close contact with the skin, and is illustrated as a circular shape in the present embodiment, but may be formed in various shapes, such as an oval, a square, or a polygon, in addition to a circular shape.

Meanwhile, a suction space (S/A) in which skin can be sucked when the beauty treatment device performs the suction function is provided inside the contact surface 112, and a component (not illustrated) for allowing skin to be suctioned through the suction space may be provided inside the main body. Since the detailed configuration for performing the suction function of the beauty treatment device is a known configuration, a detailed description therefor will be omitted.

Meanwhile, a plurality of high-frequency electrodes 182 may be disposed around the periphery of the contact surface 112 so that the beauty treatment device can apply high-frequency stimulation to the skin. Such high-frequency electrodes may stimulate the skin by allowing high-frequency current to flow while the contact surface of the beauty treatment device is in close contact with the skin.

Since the detailed configuration for applying a stimulus to cause a high-frequency current to flow to the skin is a known configuration, in the present specification, the configuration of the stimulation application unit for providing the high-frequency stimulus is explained with a focus on the description related to the contact detection unit according to the embodiment of the present disclosure, and a detailed description of the known configuration is omitted.

Meanwhile, according to one embodiment of the present disclosure, contact detection members 132 and 134 are formed to be exposed as a part of a contact detection sensor on the contact surface 112. The contact detection members 132 and 134 may be formed to have a circular protrusion protruding from the contact surface. The contact detection members 132 and 134 may be spaced apart from the high-frequency stimulation application electrode protruding from the contact surface.

In this case, referring to FIG. 2, the contact detection members 132 and 134 may be formed as a pair of a first contact detection member 132 and a second contact detection member 134.

In addition, a plurality of pairs of contact detection members may be provided on the contact surface 112.

According to one embodiment of the present disclosure, as illustrated in FIG. 2, two first contact detection members 132 and two second contact detection members 134 may be spaced apart from each other at 90 degrees.

Accordingly, the beauty treatment device having a contact detection sensor according to one embodiment of the present disclosure may be formed so that the stimulation application unit operates only when two pairs of contact detection members equipped on the contact surface are in contact with the skin.

Hereinafter, the detailed configuration and operation of the beauty treatment device 100 having a contact detection sensor will be described with reference to the following drawings.

FIG. 3 is a schematic configuration diagram of the skin beauty device according to one embodiment of the present disclosure. (a) of FIG. 4 is a graph illustrating a case where it is determined that a capacitance according to voltage supplied from the voltage supply module of the skin beauty device according to one embodiment of the present disclosure corresponds to a specific value and thus no contact has been made with the skin, and (b) of FIG. 4 is a graph illustrating a case where it is determined that the capacitance according to the voltage supplied from the voltage supply module of the skin beauty device according to one embodiment of the present disclosure does not correspond to the specific value and thus contact has been made with the skin. (a) of FIG. 5 is a graph illustrating a time interval during which a stimulation application unit applies a stimulation in the skin beauty device according to one embodiment of the present disclosure, and (b) of FIG. 5 is a graph illustrating a time interval during which the voltage supply module supplies a contact detection voltage.

Referring to FIG. 3, the beauty treatment device 100 having a contact detection sensor according to one embodiment of the present disclosure has a stimulation application unit 180, a contact detection unit 120, and a controller 170 inside the main body 110 having the contact surface 112. The main body 110 and the stimulation application unit 180 have been described above with reference to FIGS. 1 and 2, and the contact detection unit 120 and the controller 170 will be described in detail below.

Referring to FIG. 3, in one embodiment of the present disclosure, the contact detection unit 120 includes a contact detection sensor 130, a capacitor 144, a voltage supply module 150, and an AD converter 160.

The contact detection sensor 130 includes first and second contact detection members 132 and 134 installed on the contact surface as described above. In FIG. 3, a pair of contact detection members 132 and 134 is illustrated for the sake of simplicity of the drawing, but the contact detection members may be provided in multiple pairs.

As can be seen in FIG. 2, the first and second contact detection members 132 and 134 may be formed of a conductive member having a protrusion protruding from one side onto the contact surface and the other side extending into the body.

The contact detection sensor 130 is connected to the capacitor 144 and configured so that the capacitance of the capacitor 144 varies depending on whether the skin is in contact with the contact detection sensor 130.

More specifically, referring to FIGS. 2 and 3, one side of the first contact detection member 132 is provided with a protrusion exposed to the outside on the contact surface, and the other side located inside the body is formed to be grounded. That is, the first contact detection member 132 forms a ground electrode.

Meanwhile, the second contact detection member 134 is formed as a conductive member that is spaced apart from the first contact detection member 132 by 90 degrees on the contact surface, has one side having a protrusion protruding onto the contact surface, and the other side extending into the body.

The second contact detection member 134 is electrically connected to the voltage supply module 150. The voltage supply module may be a microprocessor unit (MPU).

In this case, a switching element 152 is provided between the second contact detection member 134 and the voltage supply module 150 so that voltage may be supplied according to the on/off of the switching element 152.

One side of the capacitor 144 is electrically connected to the other side of the second contact detection member 134. In this case, the other side of the capacitor 144 is formed to be grounded.

In addition, the AD converter 160 is also electrically connected to the other side of the second contact detection member 134 to convert an analog signal for a change in the capacitance of the capacitor into a digital signal.

In one embodiment of the present disclosure, the contact detection unit may be implemented as a capacitive sensor. This may be simply configured in a known capacitance voltage divider (CVD) manner to detect contact, and may detect the contact status of the skin as a change in capacitance and read the value through an AD converter, thereby simply detecting only the presence or absence of contact.

In this case, according to one embodiment of the present disclosure, as illustrated in FIG. 4, the voltage supply module 150 supplies a square wave voltage of, for example, 5 V.

When the first contact detection member 132 and the second contact detection member 134 do not come into contact with the skin, the first contact detection member 132 and the second contact detection member 134 are not electrically connected, so the voltage of 5 V, which is the initial voltage supplied by the voltage supply module 150, is applied to the capacitor 144, and the corresponding capacitance may be detected through the AD converter.

Accordingly, when it is determined that the voltage of 5 V is applied to the capacitor 144 and the corresponding capacitance is detected, the controller may determine that the state of not contacting the skin is maintained.

In this case, the voltage applied to the capacitor 144 may be the same as the initial voltage supplied by the voltage supply module, but may vary depending on the internal resistance of components inside the main body.

Meanwhile, when the first contact detection member 132 and the second contact detection member 134 come into contact with the skin even a little, current flows between the first contact detection member 132 and the second contact detection member 134, so that electricity flows through the skin of the user, and thus the skin of the user is electrically connected in parallel with the capacitor. Accordingly, the capacitance of the capacitor 144 becomes different from the capacitance of the initial capacitor 144.

In more detail, depending on the state in which the first contact detection member 132 and the second contact detection member 134 are in contact with the skin, the amount of charge that is passed between the first contact detection member 132 and the second contact detection member 134 that are grounded changes, and the capacitance of the capacitor 144 changes depending on the amount of charge that is passed.

For example, when the current flow between the first contact detection member 132 and the second contact detection member 134 is at its maximum, the voltage applied to the capacitor 144 is approximately 2.5 V as illustrated in (b) of FIG. 4, and the corresponding capacitance may be detected through the AD converter. In this case, the controller may determine that the skin treatment device has come into contact with the skin.

In this case, in (a) of FIG. 4 and (b) of FIG. 4, Ta is the time at which voltage is applied from the voltage supply module 150, and Tb means the time at which capacitance is measured by time delay.

In this case, the amount of current flowing between the first contact detection member 132 and the second contact detection member 134 may vary depending on the condition of the skin, for example, whether the skin is very sweaty, dry, or wet.

Therefore, according to one embodiment of the present disclosure, when the first contact detection member 132 and the second contact detection member 134 are completely separated from the skin, the capacitance value of the capacitor 144 according to the initial voltage supplied from the initial voltage supply module is detected, but when the first contact detection member 132 and the second contact detection member 134 come into contact with the skin even a little and are in a state where they conduct electricity with each other, it may be determined that a change occurs in the capacitance of the capacitor 144.

Therefore, according to one embodiment of the present disclosure, the contact detection unit 120 installed in the beauty treatment device 100 may be used to determine a state in which the beauty treatment device 100 is not in contact with the skin and a state in which the beauty treatment device 100 is in contact with the skin and is energized.

Meanwhile, according to one embodiment of the present disclosure, the contact detection unit 120 including the capacitor 144, the voltage supply module 150, the switching element 152, and the AD converter 160 is connected to the controller 170 inside the main body.

The controller 170 is electrically connected to the stimulation application unit 180 and controls the operation of the stimulation application unit 180 according to the capacitance of the capacitor 144 detected by the contact detection unit 120.

Referring to FIG. 5, the controller 170 of the skin treatment device according to one embodiment of the present disclosure may apply high-frequency stimulation in a time interval of 0 to t1 (first section), a time interval of t2 to t3 (third section), and a time interval of t4 to t5 (fifth section) when the stimulation applied from the stimulation application unit 180 is high-frequency current, and may apply voltage applied to the contact detection unit 120 to t1 to t2 (second section), t3 to t4 (fourth section), and t5 to t6 (sixth section).

In this way, by alternately applying the time for applying the stimulus to the stimulation application unit 180 and the time for supplying voltage to the contact detection unit 120, when there is no skin contact between the times for applying the stimulus to the stimulation application unit 180, it is possible to prevent the stimulus from being applied to the stimulation application unit 180 after there is no skin contact.

In this case, the time for applying the stimulus to the stimulation application unit 180 and the time for supplying voltage to the contact detection unit 120 may be changed by setting. For example, the time for applying the stimulus to the stimulation application unit 180 may be set to 0.9 seconds, and the time for supplying voltage to the contact detection unit 120 may be set to 0.1 seconds.

By doing this, when the beauty treatment device 100 is separated from the skin, the stimulation by the stimulation application unit 180 may be prevented from being applied to the skin, and thus, the user's safety may be improved when receiving treatment using the beauty treatment device 100.

FIG. 6 is a flowchart illustrating a method for controlling the beauty treatment device 100 according to one embodiment of the present disclosure.

Referring to FIG. 6, a method for controlling the skin beauty device according to one embodiment of the present disclosure is a method for controlling a beauty treatment device having a stimulation application unit that applies stimulation to the skin of the user by coming into contact with the skin of the user, like the skin beauty device described above, and a contact detection sensor including a capacitive sensor for detecting whether the device has come into contact with the skin of the user.

In this case, the control method for the beauty treatment device according to one embodiment of the present disclosure is as follows.

First, the main body of the beauty treatment device 100 is brought into contact with the skin (S10).

After that, it is first determined whether the main body of the beauty treatment device has come into contact with the skin (S20).

In this case, whether the main body of the beauty treatment device 100 is in contact with the skin may be determined that the main body is not in contact with the skin when the capacitance of the capacitor 144 corresponds to the specific value corresponding to the output value of the square wave voltage, for example, 5 V, as described above, and when it is measured as a value that does not correspond to the specific value or is smaller than the specific value, it may be determined that the main body is in contact with the skin. When the main body is not in contact with the skin, the step of bringing the main body of the beauty treatment device into contact with the skin is performed again.

When it is determined that the main body is in contact with the skin, the stimulation application unit applies the stimulus to the skin for a first time interval (S30). In this case, the time for applying the stimulus to the skin may be selected as a preset time.

After applying the stimulus during the first time interval, a square wave voltage is provided to measure a voltage corresponding to the capacitance of the capacitive sensor of the contact detection unit during a predetermined specified second time interval (S40).

After providing the square wave voltage in this way, a second determination is made as to whether the main body has come into contact with the skin (S50).

In this case, when it is determined that the main body is still in contact with the skin during the second determination, the stimulation is applied again during the first time interval.

In this way, the step of applying a stimulus during the first time interval and providing a square wave voltage during the second time interval may be repeated multiple times depending on the treatment time.

After the step of providing the square wave voltage, the main body is determined to be in a non-contact state in the step of second determining whether the main body is in contact with the skin, the operation of the stimulation application unit 180 is not performed and the work is terminated (S60).

After that, the main body of the beauty treatment device 100 is brought into contact with the skin again, and then it is determined whether the skin beauty device has come into contact with the skin, and the operation of the stimulation application unit may be restarted.

Meanwhile, after the step of applying the stimulus to the skin during the first time interval and the step of providing the square wave voltage to measure the capacitance of the capacitive sensor of the contact detection unit during the second time interval after the first time interval has elapsed are repeated multiple times, the step of second determining whether the main body is in contact with the skin may be performed.

According to one embodiment of the present disclosure, as described above, when the skin treatment device is separated from the skin during a predetermined skin treatment time during which stimulation is applied using the stimulation application unit, the stimulation application unit is stopped, and the skin treatment is performed while confirming whether the skin treatment device is in contact with the skin, thereby allowing the skin treatment to be performed more stably using the skin treatment device.

Although the embodiments of the present disclosure have been described, the spirit of the present disclosure is not limited to the embodiments presented in the present specification, and those skilled in the art who understand the spirit of the present disclosure will be able to easily propose other embodiments by adding, changing, deleting, or incorporating components within the scope of the same spirit, but this will also be considered to fall within the spirit of the present disclosure.

## Claims

1. A beauty treatment device having a contact detection sensor to perform a beauty treatment on a skin of a user, the beauty treatment device comprising:
a main body having a contact surface that comes into contact with the skin of the user;
a stimulation application unit provided inside the main body to apply stimulation to the skin of the user in a state where the contact surface is in contact with the skin of the user;
a contact detection unit including a contact detection sensor provided on the contact surface; and
a controller for determining whether the contact detection sensor is in contact with the skin of the user and controlling the stimulation application unit so that the stimulation application unit operates only when the contact detection sensor is in contact with the skin of the user.

2. The beauty treatment device of claim 1, wherein the stimulation applied to the skin of the user by the stimulation application unit is provided by a high-frequency electrode or light emitted to the skin.

3. The beauty treatment device of claim 1, wherein the contact surface is formed as any one of a circle, an ellipse, and a polygon,
the stimulus applied from the stimulation application unit is formed to be applied toward the skin of the user at inside the contact surface or at a location on a periphery of the contact surface, and
the contact detection sensor is positioned at a different location on the periphery of the contact surface.

4. The beauty treatment device of claim 3, wherein the contact detection sensor includes at least one pair of contact detection members disposed on the periphery of the contact surface.

5. The beauty treatment device of claim 4, wherein the contact detection member is disposed to be spaced apart at equal intervals along the periphery of the contact surface.

6. The beauty treatment device of claim 4, wherein the contact detection unit includes:
a capacitor electrically connected to the contact detection member and having a capacitance that varies depending on whether or not there is contact with the skin;
a voltage supply module that supplies a predetermined square wave voltage to the capacitor; and
an AD converter for measuring the capacitance of the capacitor.

7. The beauty treatment device of claim 6, wherein the contact detection member includes first and second contact detection members that are spaced apart from each other,
the capacitor is electrically connected to the voltage supply module and connected to the first contact detection member, and
a ground is connected in parallel to the capacitor when both the first contact detection member and the second contact detection member come into contact with the skin of the user.

8. The beauty treatment device of claim 7, further comprising a switching element for turning on and off the square wave voltage supplied from the voltage supply module.

9. The beauty treatment device of claim 8, wherein the controller determines that the skin is not in contact when it is determined that the capacitance according to the predetermined square wave voltage supplied from the voltage supply module is a specific value, and determines that the skin is in contact when the capacitance is not the specific value.

10. The beauty treatment device of claim 8, wherein the controller
provides a first time interval signal for applying the stimulus from the stimulation application unit to the stimulation application unit, and
provides a second time interval signal for supplying the square wave voltage from the voltage supply module to the voltage supply module, and
the first time interval signal and the second time interval signal are alternately and periodically provided.

11. A control method for a beauty treatment device having a contact detection sensor including a stimulation application unit that applies stimulation to a skin of a user by coming into contact with the skin of the user and a capacitive sensor for detecting whether the device has come into contact with the skin of the user, the control method comprising:
first determining whether a main body of the beauty treatment device has come into contact with the skin;
applying the stimulation to the skin for a first time interval from the stimulation application unit when the main body has come into contact with the skin;
providing a square wave voltage to measure a capacitance of the capacitive sensor of a contact detection unit for a second time interval after the first time interval has elapsed; and
second determining whether the main body has come into contact with the skin.

12. The control method of claim 11, wherein in the determining of whether the main body of the beauty treatment device is in contact with the skin,
when the capacitance of the capacitive sensor corresponds to a specific value corresponding to an output value of the square wave voltage, it is determined that the main body is not in contact with the skin, and
when the capacitance does not correspond to the specific value, it is determined that the main body is in contact with the skin.

13. The control method of claim 11, wherein after the applying of the stimulus to the skin during the first time interval and the providing of the square wave voltage to measure the capacitance of the capacitive sensor of the contact detection unit during the second time interval after the first time interval has elapsed are repeated multiple times, the second determining of whether the main body has come into contact with the skin is performed.
